Europäisches Patentamt

European Patent Office

Office européen des brevets

(11) Numéro de publication: **0 348 295**
**A1**

## DEMANDE DE BREVET EUROPEEN

(21) Numéro de dépôt: **89401734.2**

(22) Date de dépôt: **20.06.89**

(51) Int. Cl.4: **A 61 F 2/02**

(30) Priorité: **21.06.88 FR 8808689**

(43) Date de publication de la demande:
**27.12.89 Bulletin 89/52**

(84) Etats contractants désignés:
**AT BE CH DE ES FR GB GR IT LI LU NL SE**

(71) Demandeur: **LEFEBVRE, Jean-Marie**
**219, boulevard de la Liberté**
**F-59800 Lille (FR)**

(72) Inventeur: **LEFEBVRE, Jean-Marie**
**219, boulevard de la Liberté**
**F-59800 Lille (FR)**

(74) Mandataire: **Descourtieux, Philippe et al**
**CABINET BEAU de LOMENIE 55 rue d'Amsterdam**
**F-75008 Paris (FR)**

(54) **Filtre à usage médical.**

(57) L'invention est relative à un filtre à usage médical ainsi qu'à un cathéter conçu pour la mise en place et l'utilisation dudit filtre.

Selon l'invention, le filtre (1) est notamment destiné à être implanté dans un vaisseau tel que la veine cave inférieure par voie endoveineuse par l'intermédiaire d'un cathéter.

Le filtre (1) comporte des moyens de filtration (2) aptes à se déployer dans le vaisseau (3) lorsqu' il est positionné dans l'individu, lesdits moyens de filtration étant exempts de moyens d'accrochage sur la paroi veineuse.

En outre, il comporte des moyens (8) de maintien en place des moyens de filtration (2) par rapport au vaisseau (3) aptes à permettre le passage d'une situation utilisation temporaire à une situation utilisation définitive du filtre (1).

FIG.2

EP 0 348 295 A1

**Description**

## FILTRE A USAGE MEDICAL

L'invention est relative à un filtre à usage médical, notamment destiné à être implanté par voie endoveineuse, ainsi qu'à un cathéter conçu pour la mise en place et l'utilisation dudit filtre

Plus précisément , le filtre de la présente invention est un filtre à caillots destiné à être implanté , chez certains malades, par voie endoveineuse dans la veine cave inférieure pour empêcher la migration de caillots provenant des veines inférieures du corps dans le coeur et l'artère pulmonaire afin d'éviter l'embolie.

Actuellement, on connaît de tels filtres , par exemple dénommés "filtres de Greenfield" ou encore "filtres de Mobin-Uddin" US.A.3,540,431 constitués d'éléments filiformes disposés pour constituer un cône perméable au flux sanguin.

Pour assurer la fixation du filtre dans la veine, généralement les extrémités des éléments filiformes constituant les moyens de filtration sont pourvues de crochets d'amarrage venant se piquer dans la paroi interne de la veine, comme cela est décrit dans les documents US.A.4,425,908 , FR.A.2.587.901 et EP.A.0.270.432.

Pour implanter de tels filtres , généralement , on utilise la voie endoveineuse ; plus précisément , on introduit le filtre par une petite veine telle que la veine jugulaire ou la veine fémorale , puis on guide et on conduit le filtre vers la veine cave inférieure , là où il se fixera d'une manière stable en se dépliant.

Pour faciliter le cheminement , on utilise un cathéter qui, selon les dimensions , est introduit lors d'une intervention chirurgicale ou en mettant en oeuvre une méthode d'introduction percutanée moins agressive pour le patient Cela étant , la grande majorité des filtres existants sont "définitifs" , c'est-à-dire, qu'une fois implantés , ils ne peuvent plus être ôtés du patient.

En effet, la structure de ces filtres est telle que les moyens de fixation du filtre , formés de crochets d'amarrage, se piquent ou s'agrippent dans la paroi interne de la veine, ce qui interdit tout cheminement inverse.

Cependant , certains patients ne présentent un risque d'embolie pulmonaire que pendant une brève période. En effet, par exemple , pendant une période opératoire , ou lorsque les patients sont porteurs de caillots périphériques , lesdits caillots peuvent être dissous rapidement par des médicaments fibrinolytiques, ce qui ne justifie pas l'insertion d'un filtre à caillots d'une manière définitive.

C'est pourquoi , il est intéressant de disposer de filtres pour la veine cave aptes à être mis en place d'une manière temporaire c'est-à-dire aptes à n'être laissés en place que quelques jours par exemple.

En effet , dans de tels cas , sur le plan clinique, deux évolutions sont possibles , à savoir dissolution et disparition complète des caillots , ou alors aggravation du risque et persistance des caillots.

En ce qui concerne les filtres à usage temporaire, on utilise de nos jours des filtres de confection semblable aux filtres définitifs , néanmoins , ceux-ci ne portent pas de crochets d'amarrage pour permettre leur retrait par cheminement inverse par voie endoveineuse. Le document FR.A.2.580.504 décrit un filtre temporaire de ce type.

Pour autoriser la fixation des filtres temporaires connus, c'est-à-dire pour éviter sa migration dans la veine, on utilise généralement un guide fixé d'une part au filtre temporaire et qui d'autre part chemine dans le vaisseau sanguin jusqu'au point de ponction, où là , il est maintenu en place par exemple par points de suture.

Dans le cas où l'évolution est satisfaisante , c'est-à-dire lorsque les contrôles radiographiques permettent d'apprécier que l'état des veines et de la veine cave s'est normalisé et que les risques d'embolie ont disparu , il est intéressant de pouvoir ôter le filtre.

Par contre, si la situation reste préoccupante et le risque d'embolie demeure important , il est nécessaire de maintenir en place le filtre.

Or , si un filtre temporaire a été utilisé, il est alors nécessaire de le remplacer par un filtre définitif , ce qui implique de réintervenir de nouveau , soit en faisant une nouvelle opération pour mettre en place un autre filtre , soit en remplaçant le filtre temporaire en place par un nouveau filtre définitif , obligeant à réintroduire des cathéters et des guides métalliques au niveau de la zone de ponction , ce qui présente un risque important sur le plan de la contamination infectieuse.

L'évolution incertaine sur le plan clinique et la maîtrise des risques d'embolie étant délicates , il serait intéressant de disposer d'un filtre temporaire et/ou définitif , pouvant, après quelques jours , soit être retiré , soit être laissé en place définitivement sans qu'il ne soit nécessaire d'introduire à l'intérieur du système veineux du patient , à nouveau, des cathéters ou des guides métalliques.

Le but de la présente invention est de proposer un tel filtre à usage médical , notamment destiné à être implanté dans un vaisseau , tel que la veine cave inférieure , par voie endoveineuse par l'intermédiaire d'un cathéter , qui permette de pallier les inconvénients des filtres connus.

Autrement dit , un des buts de la présente invention est de proposer un filtre à usage médical , à usage temporaire et / ou définitif , qui puisse , soit être retiré sans préjudice pour le système veineux du patient , soit être laissé définitivement en place en évitant une nouvelle intervention qui serait préjudiciable compte-tenu des risques de contamination infectieuse.

Un autre but de la présente invention est de proposer un filtre à usage médical , implantable par voie endoveineuse dans la veine cave , dont la fixation définitive est renforcée pour éviter la migration du filtre lors de mouvements péristaltiques.

Un autre but de la présente invention est de proposer un cathéter conçu pour la mise en place et l'utilisation du filtre de l'invention qui autorise notamment son implantation par voie endoveineuse percutanée.

Un autre but de la présente invention est de proposer un cathéter qui permette le contrôle et le maintien dudit filtre en utilisation temporaire.

En outre, ledit cathéter de la présente invention permet l'introduction en amont du filtre de produit fibrinolytique pour lyser les caillots qui peuvent se former ou être piégés dans le filtre, et pour pouvoir contrôler , grâce à l'injection de produit de contraste , une existence de caillots au niveau de la zone filtrante.

Un autre but de la présente invention est de proposer un cathéter qui permette , soit de retirer très facilement le filtre, soit de le laisser en place définitivement sans qu'il soit nécessaire de réintervenir opératoirement.

Un autre but de la présente invention est de proposer un cathéter de ce type dont le fonctionnement est parfaitement fiable malgré le risque d'accumulation de fibrine.

Un autre but de la présente invention est de proposer un filtre destiné à être implanté dans un vaisseau qui, en position définitive ou temporaire , présente une bonne fonction de filtration, ce de manière non agressive pour les parois internes du vaisseau.

D'autres buts et avantages de la présente invention apparaîtront au cours de la description qui va suivre , qui n'est cependant donnée qu'à titre indicatif et qui n'a pas pour but de la limiter.

Selon la présente invention , le filtre à usage médical, notamment destiné à être implanté dans un vaisseau tel que la veine cave inférieure , par voie endoveineuse par l'intermédiaire d'un cathéter , ledit filtre comprenant des moyens de filtration aptes à se déployer dans ledit vaisseau lorsqu' il est positionné dans l'individu , est caractérisé par le fait que les moyens de filtration sont exempts de moyens d'accrochage sur la paroi veineuse et par le fait qu'il comporte des moyens de maintien en place des moyens de filtration par rapport au vaisseau , aptes à permettre le passage d'une situation utilisation soit temporaire, c'est-à-dire qu'étant maintenu pendant un certain temps le filtre pourra être retiré sans dommage pour le vaisseau, soit définitive , c'est-à-dire que le filtre pourra être laissé sur place, solidement accroché sur la paroi du vaisseau.

En outre , le cathéter conçu pour la mise en place et l'utilisation du filtre de l'invention , présentant une extrémité proximale et une extrémité distale , ledit filtre étant préalablement à l'implantation dans l'individu placé au niveau de l'extrémité distale du cathéter est caractérisé par le fait qu'il comporte :
- une gaine de descente dont l'extrémité distale est apte d'une part à maintenir non déployés les moyens de filtration pendant l'introduction du filtre et d'autre part à contenir fixement les moyens de maintien pendant l'introduction du filtre, son utilisation temporaire et son retrait après utilisation temporaire,
- un poussoir manoeuvrable à partir de l'extrémité proximale du cathéter , apte , pour l'utilisation définitive du filtre , à expulser lesdits moyens de maintien hors de la gaine de descente au niveau de son extrémité distale.

On comprend que contrairement à ce qui est enseigné dans les brevets précités décrivant des filtres définitifs , les moyens de filtration doivent impérativement être exempts de moyens d'accrochage sur la paroi veineuse , de manière à ce qu'en cas de retrait du filtre les moyens de filtration puissent glisser à l'intérieur de la veine sans l'endommager.

La présente invention sera mieux comprise à la lecture de la description suivante accompagnée des dessins en annexe qui en font partie intégrante.

La figure 1 illustre un mode de réalisation du filtre de la présente invention vu en perspective.

La figure 2 représente le filtre de la figure 1 vu en plan, implanté dans un vaisseau sanguin.

La figure 3 montre une vue en coupe d'un cathéter selon la présente invention équipé dudit filtre.

Les figures 4a à 4d montrent l'implantation du filtre de l'invention , par l' intermédiaire du cathéter représenté à la figure 3, dans une phase d'utilisation temporaire.

Les figures 5a et b montrent le passage de la situation utilisation temporaire à la situation utilisation définitive du filtre de la présente invention.

La figure 6 montre une variante de réalisation d'un filtre et du cathéter selon la présente invention.

La présente invention vise un filtre à usage médical, implantable dans un vaisseau ainsi qu'un cathéter conçu pour la mise en oeuvre dudit filtre.

Le filtre de la présente invention trouvera notamment son application pour constituer un filtre à caillots dans la veine cave inférieure pour pallier les risques d 'embolie pulmonaire chez un patient.

Sur le plan clinique , dans de nombreux cas ,les risques sont bien souvent temporaires , mais hélas peuvent persister et , dans ce cas , le risque d'embolie est très important.

A l'opposé des filtres connus qui sont soit temporaires, soit définitifs , le concept du filtre de la présente invention permet son utilisation temporaire et/ou définitive , c'est-à-dire qu'il peut être après quelques jours , soit retiré , soit laissé en place définitivement sans intervention supplémentaire traumatisante pour le patient.

De plus , le filtre temporaire et/ou définitif de la présente invention respecte les critères nécessaires dans ce cas , à savoir : mise en place facile par voie percutanée , bonne fonction de filtration , bonne fonction de fixation.

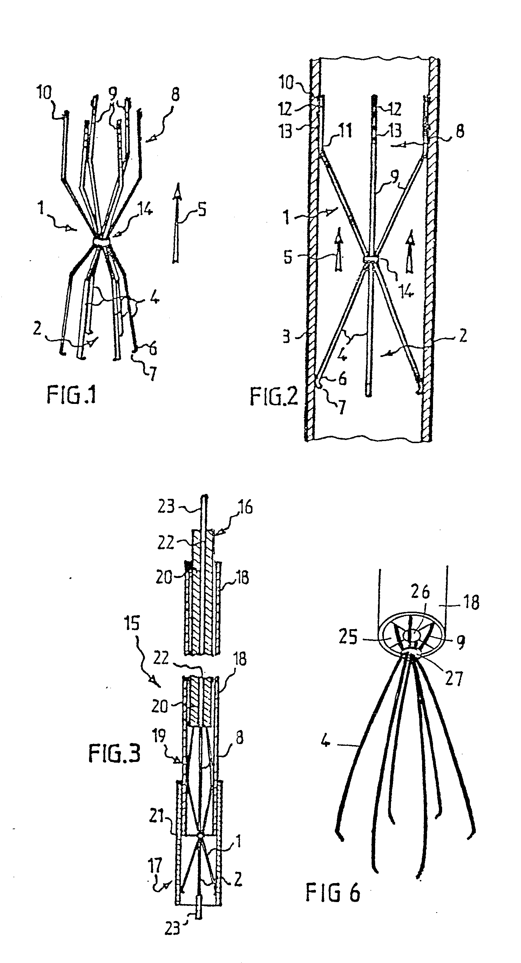

Comme le montrent les figures 1 et 2, le filtre 1 de la présente invention comporte tout d'abord des moyens de filtration 2 aptes à se déployer dans le vaisseau sanguin 3, notamment la veine cave inférieure , lorsque le filtre est correctement positionné dans l'individu.

Ces moyens de filtration 2 sont constitués avantageusement par un ensemble d'éléments filiformes 4, flexibles , réunis entre eux par une de leurs extrémités pour former sensiblement l'enveloppe d'un cône , ogive ou similaire.

Ainsi , cet ensemble d'éléments filiformes 4 de filtration sera perméable au flux sanguin, repéré par les flèches 5 sur les figures , mais apte à retenir les caillots. Pour ce , il sera disposé dans le sens du flux sanguin c'est-à-dire que la pointe du cône ou similaire sera dirigée sensiblement dans le même sens que le flux sanguin.

A titre d'exemple , les tiges 4 flexibles seront au nombre de six et réalisées dans un matériau bio-compatible tel qu'en alliage métallique ou en matière plastique adaptée. Toutefois , ces matières seront choisies pour leur aptitude à l'élasticité et à la déformation.

En effet , les tiges 4 représenteront une bonne flexibilité pour d'une part être repliées dans un cathéter de faible dimension et d'autre part se déployer correctement dans le vaisseau sanguin pour former un barrage aux dimensions de celui-ci.

Par ailleurs , les éléments filiformes de filtration 4 seront tels qu'ils soient non agressifs vis-à-vis des parois internes du vaisseau.

Plus précisément , comme le montrent les figures 1 et 2, les tiges 4 présentent au moins une légère angulation ou courbure 6 telle que leur partie terminale 7 soit en contact non agressif avec le vaisseau , lorsque les moyens de filtration sont déployés, mais de façon qu'il n'y ait pas pénétration des extrémités des tiges 4 dans la paroi du vaisseau proprement dit.

La mise en oeuvre de ces moyens de filtration 4 sera décrite plus en détail avec la description dti cathéter et du procédé d'implantation du filtre décrit ultérieurement.

Cela étant , le filtre à usage médical 1 de la présente invention comporte des moyens 8 de maintien en place, par rapport au vaisseau 3, des moyens de filtration 2, aptes à permettre le passage d'une situation utilisation temporaire à une situation utilisation définitive du filtre 1.

Plus précisément , comme le montrent notamment les figures 1 et 2, ces moyens 8 de maintien sont dissociés et placés en aval desdits moyens de filtration 2. Ils sont aptes , d'une part à être repliés et contrôlés de l'extérieur de l'individu pour autoriser le maintien du filtre 1 en utilisation temporaire, indépendamment du vaisseau 3, et d'autre part aptes à être déployés pour permettre l'accrochage du filtre 1 au niveau des parois internes du vaisseau 3 en utilisation définitive.

Plus précisément , comme le montre un mode de réalisation de la présente invention , illustré aux figures 1 et 2 , lesdits moyens de maintien 8 sont constitués par un ensemble d'éléments filiformes 9 flexibles , réunis entre eux par une de leurs extrémités pour former sensiblement l'enveloppe d'un cône ou d'une ogive ou similaire, perméable au flux sanguin 5.

Les différentes tiges 9 de fixation sont réalisées en matière bio-compatible telle qu'en plastique ou en alliage métallique adapté. De tels matériaux permettent par exemple une bonne flexibilité et autorisent en outre la réalisation de diverses formes requises pour la fixation.

En particulier , les extrémités 10 desdits éléments filiformes de maintien 9, qui définissent sensiblement la base dudit cône ou similaire , sont acérées afin de pouvoir s'agripper sur les parois internes du vaisseau 3. A titre d'exemple , on a représenté sur la figure 2 de telles extrémités acérées 10 en forme de crochets.

En outre , pour augmenter encore l'efficacité des parties terminales 12 , on prévoira avantageusement des aspérités 13, telles que notamment des ergots , dirigés vers la paroi du vaisseau, ayant tendance à pénétrer dans la paroi grâce à la pression précitée exercée.

La combinaison de ces trois éléments , à savoir , extrémités acérées 10, partie terminale 12 et aspérités 13, procurera au filtre de la présente invention une très bonne stabilité en position utilisation définitive et permettra d'éviter toute migration du filtre à plus ou moins long terme due au mouvement péristaltique du vaisseau.

Dans le mode de réalisation représenté sur les figures , l'ensemble 8 d'éléments filiformes de maintien 9 et l'ensemble 2 d'éléments filiformes de filtration 4 sont disposés tête bêche et réunis par le sommet de chaque ensemble.

Toutefois , cette disposition n'est nullement impérative et on pourrait envisager le cas où les deux cônes 2 et 8 seraient dirigés dans le même sens , à savoir le sens imposé par le flux sanguin 5, et décalés l'un par rapport à l'autre afin que l'espace de filtration et l'espace de fixation soient dissociés.

Par ailleurs , pour en terminer avec la structure du filtre de la présente invention , deux autres détails de structure peuvent être envisagés pour faciliter d'une part la mise en place et d'autre part faciliter le travail du praticien et /ou augmenter l'efficacité du traitement.

En ce qui concerne ce dernier point , la zone centrale du filtre , à savoir , dans le mode de réalisation représenté sur les figures , la zone de jonction des deux ensembles 8 et 2 d'éléments filiformes de maintien 9 et de filtration 4, présente un orifice 14 qui permettra avantageusement le passage d'un cathéter de perfusion 22, 23 , apte à véhiculer un liquide.

Ainsi , par l'intermédiaire du filtre de la présente invention, on pourra introduire avantageusement en amont du filtre 1, du produit fibrinolytique pour lyser les caillots qui peuvent se former ou être piégés par les moyens de filtration 2.

En outre , on pourrait également , pour faciliter la localisation des caillots , perfuser par l'intermédiaire de ce cathéter une solution de contraste en amont de la zone de filtration.

Enfin , pour autoriser une mise en oeuvre plus souple des moyens de fixation 8, et par suite faciliter la mise en place du filtre , les éléments filiformes de maintien 9 présenteront des longueurs différentes les unes par rapport aux autres. Ainsi, lors du lâcher du filtre , on aura un déploiement progressif des moyens de fixation 8 du filtre 1.

Cela étant , pour autoriser la mise en place et l'utilisation du filtre , tel que notamment décrit en regard des figures 1 et 2 , la présente invention propose un cathéter adapté.

Un mode de réalisation schématique d'un tel cathéter est illustré à la figure 3. Ledit cathéter 15 présente une extrémité proximale 16 et une extrémité distale 17 au niveau de laquelle ledit filtre 1 est préalablement placé.

Le cathéter 15 comporte au moins :
- une gaine principale de descente 18, constituant le corps principal du cathéter 15, dont l'extrémité distale 19 est apte d'une part à contenir lesdits moyens de maintien 8 du filtre 1, et d'autre part à maintenir non déployés lesdits moyens de filtration 2 du filtre pendant l'introduction. de ce dernier;
- un poussoir 20 , manoeuvrable à partir de l'extrémité proximale 16 du cathéter , apte à expulser lesdits moyens de maintien 8 hors de la gaine de descente 18, au niveau de son extrémité distale 19.

En outre , pour maintenir les moyens de filtration 2 repliés , lors de la phase d'introduction du cathéter 15, une première solution consisterait à les intégrer dans la zone intérieure distale 19 de la gaine de descente 18.

Une deuxième solution consiste à prévoir , au niveau de l'extrémité distale 19 de la gaine de descente 18, un manchon coulissant 21 apte d'une part à contenir lesdits moyens de filtration 2 repliés et d'autre part à coulisser sur la gaine extérieure 18 jusque l'extrémité proximale 16 du cathéter.

Enfin , pour autoriser la délivrance de produit fibrinolytique ou de produit de contraste , en amont du filtre , le cathéter 15 comporte un conduit 22, apte à véhiculer ledit liquide en amont du filtre.

Pour ce, ledit conduit 22 s'étend de l'extrémité proximale 16 à l'extrémité distale 17 coaxialement à la gaine 18 et au filtre 1.

Pratiquement , ce conduit 22 est formé d'une lumière coaxiale au cathéter à l'intérieur de laquelle est introduit un cathéter complémentaire du type multi-perforé dépassant en amont (du filtre. Pour des commodités de représentation , ce cathéter 23 n'est figuré , sur la figure 3, qu'au niveau de la zone proximale 16 et distale 17.

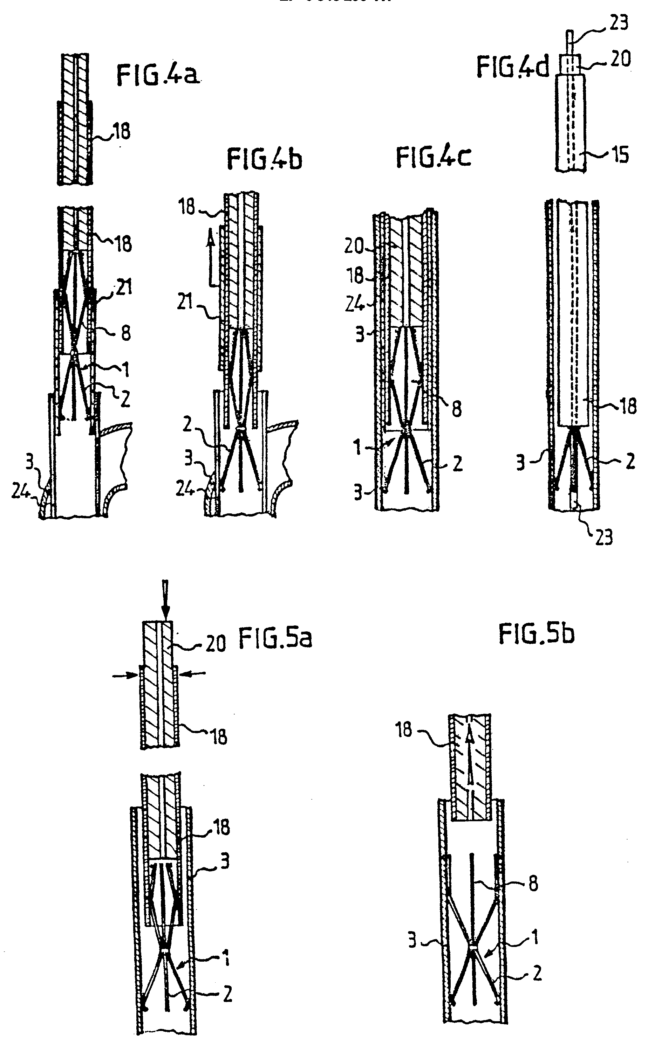

Le mode de mise en place du filtre de la présente invention à l'aide du cathéter décrit ci-dessus est illustré aux figures 4a à 4d qui , pour les besoins de la compréhension , ne respecte pas les dimensions relatives des différents éléments . Il faut se rappeler néanmoins que le cathéter est suffisamment fin pour qu'il puisse être introduit par voie percutanée de façon traditionnelle.

En effet , la procédure consiste à ponctionner la veine jugulaire interne , à introduire dans 1 'aiguille un guide métallique de longueur adaptée permettant de descendre dans la veine cave inférieure puis de faire cheminer , sur ce guide métallique , un système d'introduction constitué d'un introducteur proprement dit, non représenté sur les figures , et de sa gaine pelable, repérée 24 sur les figures 4.

Quand le système d'introduction est arrivé en bonne position, en-dessous des veines rénales , on retire le guide métallique et l'introducteur. Par contre , la gaine pelable 24 est laissée en place.

Le cathéter 15 de la présente invention est alors introduit dans la gaine pelable 24 comme le montre particulièrement la figure 4a.

Le filtre 1 est conditionné en position complètement replié dans la gaine de descente 18, les moyens de maintien 8 étant repliés à l'intérieur de l'extrémité distale 19 de la gaine 18, et les moyens de filtration 2 étant quant à eux à l'extérieur de la partie distale 19 de la gaine mais maintenus repliés par le manchon coulissant 21, en saillie pour l'instant par rapport à l'extrémité distale de la gaine 18.

Par ailleurs , à l'intérieur dudit cathéter 15, descend jusqu'à l'intérieur du filtre 1, le cathéter de perfusion 22,23, non représenté sur la figure pour ne pas la surcharger.

La figure 4b montre la phase suivante de l'introduction du cathéter 15, et particulièrement montre l'introduction des moyens de filtration 2 dans la gaine pelable 24.

Une fois les moyens de filtration 2 introduits en totalité dans la gaine pelable 24, on peut faire coulisser le manchon 21 sur la gaine de façon à ce que les moyens 2 débouchant alors de ladite gaine 18 sont maintenus repliés par la gaine pelable d'introduction 24.

La figure 4c montre l'issue de la phase d'introduction du cathéter 15, c'est-à-dire sensiblement la coïncidence de la partie distale de la gaine de descente 18 et la partie distale de la gaine pelable 24.

A ce niveau, le filtre ou plus précisément les moyens de filtration 2 du filtre sortent de la gaine pelable 24, se déploient, et assurent leur fonction de filtration.

La gaine pelable 24 peut alors être retirée , notamment par déchirement , le filtre étant alors en position temporaire comme le montre la figure 4d, moyens de filtration 2 déployés et moyens de maintien 8 repliés dans la gaine 18.

Pendant cette phase d'utilisation temporaire le cathéter de perfusion 23 pourra avantageusement être utilisé , rappelons-le, soit pour diffuser en amont du filtre un produit opaque pour contrôler l'existence de caillots au niveau de la zone filtrante 8, soit pour perfuser des produits fibrinolytiques pour lyser les caillots piégés dans cette zone.

Dans cette phase d'utilisation, le filtre est maintenu en place par le cathéter 15 grâce aux moyens de maintien 8 qui sont repliés dans l'extrémité distale intérieure 19 de la gaine de descente 18, le frottement entre ces deux éléments étant tel que le filtre et par suite les moyens de maintien ne puissent pas s'échapper du cathéter. Autrement dit, la gaine de descente 18 est dans un matériau tel que le coefficient de frottement entre les moyens de maintien et la gaine est supérieur au coefficient de frottement entre les moyens de filtration 2 et le vaisseau sanguin

Cela étant , dans cette phase dite temporaire , pour autoriser le maintien en place des moyens de filtration 2 par rapport au vaisseau 3 , le cathéter 15 sera avantageusement fixé selon des procédés traditionnels au niveau du point de ponction, par exemple par points de suture dans le muscle ou tout dispositif adapté.

Après la phase de surveillance , pendant laquelle le praticien surveille l'évolution sur le plan clinique, deux possibilités s'offrent à lui, soit le retrait du filtre, soit l'implantation définitive de ce dernier.

Lorsque le praticien décide de retirer le filtre, il suffit alors de supprimer la fixation cutanée, notamment en sectionnant les fils de suture et de remonter l'ensemble. pour ressortir le filtre et les moyens de filtration par l'orifice de ponction.

Par contre, si la situation clinique reste préoccupante et les risques d'embolie demeurent importants, il sera nécessaire de laisser en place définitivement le filtre 1.

Les figures 5a et 5b montrent cette procédure par laquelle on éjecte le filtre 1 de la gaine 18 du cathéter 15.

Plus précisément , on vient mettre en saillie par rapport à la gaine 18, les moyens de fixation 8 par un mouvement relatif du poussoir 20 par rapport à la gaine 18.

Une manoeuvre , utilisant le poussoir comme un butoir en remontant la gaine pour libérer le filtre , à la manière d'un doigt de gant, représente la manoeuvre la plus adaptée pour laisser le filtre dans la position décidée précédemment. Néanmoins, d'autres dispositions pourraient être adoptées.

Ainsi, le poussoir 20 permet d'éjecter définitivement le filtre de la gaine et les éléments flexibles 9 des moyens de fixation 8 de par leurs tailles différentes , permet lorsque l'on retire la gaine de les libérer progressivement pour un accrochage plus précis.

La figure 5b illustre la mise en place définitive des filtres et le retrait de la gaine 18 du cathéter 15.

Dans certains cas, en particulier en cas d'utilisation temporaire prolongée, il peut se produire une accumulation de fibrine à l'intérieur du cathéter autour des éléments filiformes de maintien 9, de telle sorte que lors de l'expulsion du filtre, ces éléments filiformes 9 risquent d'être bloqués en position repliée et de ne pas se déployer parfaitement.

Pour pallier cet inconvénient , le cathéter ou au moins l'extrémité distale de la gaine 18 présente longitudinalement des logements indépendants 25, dans chacun desquels lors du positionnement du filtre sur le cathéter est enfilé un élément filiforme 9. La figure 6 représente un filtre selon l'invention dont les moyens de filtration sont composés de six éléments filiformes 4 et les moyens de maintien de quatre éléments filiformes 9 ; chaque élément filiforme 9 de maintien est positionné dans son logement 25 ; l'extrémité distale de la gaine 18 comporte en outre un cinquième logement cylindrique et axial 26. Ce logement 26 permet le passage du poussoir entre les quatre logements 25 et donc les éléments filiformes de maintien 9; lors du lâcher du filtre , le poussoir prend appui sur le rivet 27 assurant la jonction entre les éléments de filtration et les éléments de maintien.

Cette forme particulière du cathéter présente comme avantage complémentaire d'éviter l'emmêlement de ces éléments filiformes de maintien 9 qui était parfois constaté lorsqu' ils étaient placés tous ensemble dans le cathéter , sans compartimentation.

Naturellement , d'autres mises en oeuvre de la présente invention, à la portée de l'homme de l'art , auraient pu être envisagées sans pour autant sortir du cadre de celle-ci.

## Revendications

1. Filtre à usage médical , notamment destiné à être implanté dans un vaisseau tel que la veine cave inférieure, par voie endoveineuse par l'intermédiaire d'un cathéter (15), ledit filtre (1) comprenant des moyens de filtration (2) aptes à se déployer dans ledit vaisseau (3) lorsqu'il est positionné dans l'individu, caractérisé par le fait que les moyens de filtration sont exempts de moyens d'accrochage et par le fait qu'il comporte des moyens (8) de maintien en place desdits moyens de filtration (2) par rapport au vaisseau (3) aptes à permettre le passage d'une situation "utilisation temporaire" à une situation "utilisation définitive" du filtre (1).

2. Filtre selon la revendication 1, caractérisé par le fait que lesdits moyens (8) de maintien sont dissociés en aval desdits moyens de filtration (2) et sont d'une part aptes à être reliés et contrôlés de l'extérieur de l'individu pour autoriser le maintien du filtre (1) en utilisation temporaire indépendamment du vaisseau (3), et d'autre part aptes à être déployés pour permettre l'accrochage du filtre (1) au niveau des parois internes du vaisseau (3) en utilisation définitive.

3. Filtre selon la revendication 1, caractérisé par le fait que lesdits moyens (8) de maintien sont constitués par un ensemble d'éléments filiformes (9) flexibles , réunis entre eux par une de leurs extrémités, pour former sensiblement l'enveloppe d'un cône , d'une ogive ou similaire , perméable au flux sanguin (5).

4. Filtre selon la revendication 3 , caractérisé par le fait que les extrémités (10) des éléments filiformes de maintien (9) sont acérées.

5. Filtre selon la revendication 3 , caractérisé par le fait que lesdites parties terminales (12) de maintien présentent des aspérités (13) dirigées vers la paroi du vaisseau (3) pour éviter la migration du filtre (1) en utilisation définitive.

6. Filtre selon la revendication 3, caractérisé par le fait que lesdits éléments (9) filiformes de maintien présentent des longueurs différentes les unes par rapport aux autres.

7. Filtre selon la revendication 1, caractérisé par le fait que lesdits moyens de filtration (2) sont constitués par un ensemble d'éléments filiformes (4) flexibles , réunis entre eux par une de leurs extrémités , pour former sensiblement l'enveloppe d'un cône, ogive ou similaire , perméable au flux sanguin mais apte à retenir les caillots , disposés dans le sens du flux sanguin (5).

8 . Filtre selon la revendication 7, caractérisé par le fait que les éléments filiformes de filtration (4) présentent au moins une légère angulation ou courbure (6) telle que leur partie terminale (7) soit en contact , en position utilisation temporaire et/ou définitive , de façon non agressive vis-à-vis des parois internes du vaisseau (3).

9 . Filtre selon les revendications 3 et 7, caractérisé par le fait que lesdits ensemble (8) d'éléments filiformes de maintien (9) et ensemble (2) de filtration (4) sont disposés tête bêche et réunis par leur sommet.

10. Filtre selon la revendication 9, caractérisé par le fait que la zone de jonction (14) desdits ensembles d'éléments filiformes (8,2) présente un orifice permettant le passage d'un cathéter de perfusion (23) apte à véhiculer un liquide.

11. Cathéter conçu pour la mise en place et l'utilisation d'un filtre selon l'une quelconque des revendications précédentes, ledit cathéter (15) présentant une extrémité proximale (16) et une extrémité distale (17) , ledit filtre (1) étant préalablement à l'implantation dans l'individu placé au niveau de l'extrémité distale (17), caractérisé par le fait qu'il comporte :
- une gaine de descente dont l'extrémité distale est apte d'une part à maintenir non déployés les moyens de filtration pendant l'introduction du filtre et d'autre part à contenir fixement les moyens de maintien (8) pendant l'introduction du filtre (1), son utilisation temporaire et son retrait après utilisation temporaire,
- un poussoir manoeuvrable (20) à partir de l'extrémité proximale du cathéter et apte à expulser les moyens de maintien (8) hors de la gaine de descente (18) au niveau de son extrémité distale en cas d'utilisation définitive.

12. Cathéter selon la revendication 11, ledit cathéter (15) cheminant dans le vaisseau sanguin (3) par l'intermédiaire d'une gaine pelable d'introduction (24) préalablement placée dans ledit vaisseau sanguin (3), caractérisé par le fait que ladite gaine de descente (18) présente à son extrémité distale (19) un manchon coulissant (21) apte d'une part à contenir lesdits moyens de filtration (2) repliés et d'autre part à coulisser sur la gaine (18) jusque son extrémité proximale (16) , lesdits moyens de filtration (2) débouchant en saillie alors de ladite gaine (18) et étant maintenus repliés dans ladite gaine pelable d'introduction (24).

13. Cathéter selon la revendication 11, caractérisé par le fait qu'il comporte un conduit (22,23) apte à véhiculer un liquide en amont dudit filtre (1), ledit conduit (22,23) s'étendant de l'extrémité proximale (16) à l'extrémité distale (17) du cathéter (15) coaxialement à la gaine (18) et au filtre (1).

14. Cathéter selon la revendication 11 , caractérisé en ce que la gaine de descente (18) ou son extrémité distale (19) est compartimentée longitudinalement en au moins autant de logements indépendants (25) que d'éléments filiformes de maintien (9), chacun de ceux-ci étant apte à être enfilé dans un logement (25) correspondant.

15. Cathéter selon la revendication 14 caractérisé en ce que la gaine de descente (18) ou son extrémité distale (19) comporte un logement central (26) , dans lequel peut coulisser le poussoir (20) d'expulsion du filtre (1).

16. Cathéter selon la revendication 11 caractérisé en ce que la gaine de descente est dans un matériau tel que le coefficient de frottement entre la gaine et les moyens de maintien est supérieur au coefficient de frottement entre les moyens de filtration et le vaisseau sanguin.

EP 0 348 295 A1

FIG.1

FIG.2

FIG.3

FIG 6

FIG.4a

FIG.4b

FIG.4c

FIG.4d

FIG.5a

FIG.5b

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | US-A-4 425 908  (M. SIMON)<br>* colonne 5, lignes 34-47; colonne 6, ligne 10 - colonne 7, ligne 7; colonne 8, ligne 20 - colonne 9, ligne 31; revendications 1-5,8; figures 1-4,10-13 *<br>--- | 1,3-9, 11 | A 61 F   2/02 |
| A,D | EP-A-0 270 432  (PROMED)<br>* colonne 2, ligne 52 - colonne 3, ligne 27; colonne 4, lignes 4-37; colonne 5, lignes 2-13; revendications 1-6; figures 1-5 *<br>--- | 1,3-5,7 ,8,11 | |
| A,D | FR-A-2 587 901  (H. BOCQUEE et al.)<br>* page 1, ligne 29 - page 3, ligne 3; page 3, ligne 19 - page 4, ligne 34; revendications 1,2; figure 1-3 *<br>--- | 1,3-9, 11 | |
| A,D | FR-A-2 580 504  (A. PIERONNE)<br>* page 7, lignes 8-30; page 9, lignes 25-33; revendications 6,7,9,10; figures 1-3 *<br>----- | 1,11-13 | |
| | | | DOMAINES TECHNIQUES RECHERCHES (Int. Cl.4)<br><br>A 61 F<br>A 61 M<br>A 61 B |

Le présent rapport a été établi pour toutes les revendications

| Lieu de la recherche | Date d'achèvement de la recherche | Examinateur |
|---|---|---|
| BERLIN | 25-08-1989 | MONNE E.M.B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................
& : membre de la même famille, document correspondant

EPO FORM 1503 03.82 (P0402)